# FASCICULE DE BREVET EUROPEEN

(11) **EP 0 789 681 B1**
(45) Date de publication et mention de la délivrance du brevet: **08.09.1999**
(21) Numéro de dépôt: 95939319.0
(22) Date de dépôt: 03.11.1995
(51) Int. Cl.: C07C 51/12

(54) **PROCEDE DE PREPARATION D'ACIDES CARBOXYLIQUES PAR CARBONYLATION EN PRESENCE DE RHODIUM**
VERFAHREN ZUR HERSTELLUNG VON CARBONSÄUREN DURCH CARBONYLIERUNG IN GEGENWART DES RHODIUMS
METHOD FOR PREPARING CARBOXYLIC ACIDS BY CARBONYLATION IN THE PRESENCE OF RHODIUM

(30) Priorité: 04.11.1994 FR 9413176
(43) Date de publication de la demande: 20.08.1997
(73) Titulaire: BP Chemicals Limited, London EC2M 7BA (GB)
(72) Inventeur: DENIS, Philippe, F-69150 Décines (FR); PERRON, Robert, F-69390 Charly (FR); SCHWARTZ, Joel, F-69300 Caluire (FR)
(74) Mandataire: Perkins, Nicholas David
(86) Numéro de dépôt international: FR9501447
(87) Numéro de publication internationale: WO9614287

(56) Documents cités:
- EP-A- 0 441 260
- US-A- 3 769 329

## Description

La présente invention a pour objet un procédé de préparation d'acides carboxyliques par carbonylation d'un réactif, choisi notamment parmi les alcools, en présence d'un catalyseur à base de rhodium.

Les procédés de carbonylation mettant en oeuvre des catalyseurs solubles à base de rhodium pour la préparation d'acides carboxyliques sont des procédés bien connus. Ils sont en général mis en oeuvre dans des installations comprenant essentiellement trois zones. La première correspond à la zone de réaction proprement dite, comprenant un réacteur sous pression, dans lequel la carbonylation est effectuée. La seconde est constituée par une zone de séparation de l'acide formé. Cette opération est réalisée par vaporisation partielle du mélange réactionnel dans un appareil (appelé flash) où la pression est maintenue plus faible que dans le réacteur. La partie vaporisée est ensuite envoyée dans une troisième zone où l'acide carboxylique produit est purifié. Celle-ci comprend diverses colonnes de distillation dans lesquelles l'acide carboxylique produit est séparé de l'eau, des réactifs et des sous-produits. La partie du mélange restée sous forme liquide en sortie de la zone de vaporisation et comprenant notamment le catalyseur, est recyclée au réacteur. Ceci est classiquement réalisé au moyen d'une pompe.

Lors de la mise en oeuvre de ce type de procedé, il a fallu faire face à un problème de stabilité du catalyseur. Plus particulièrement on a constaté que le catalyseur précipitait dans la zone de vaporisation partielle du mélange réactionnel.

Plusieurs solutions ont alors été envisagées pour résoudre ce problème.

La première solution préconisée a été de maintenir une teneur élevée en eau dans le réacteur, aux alentours de 14 à 15 %. Cependant, cette solution n'est pas avantageuse sur le plan économique. En effet, l'augmentation de la teneur en eau dans le mélange réactionnel a pour conséquence d'augmenter le coût de la séparation ultérieure de l'acide produit et de l'eau.

La seconde solution envisagée revient à introduire un élément dans le mélange réactionnel afin de stabiliser le catalyseur. Ces agents de stabilisation sont notamment choisis parmi les iodures de métaux alcalins, tels que l'iodure de lithium ou encore l'iodure de potassium, ou bien encore choisis parmi des composés organiques susceptibles de former des espèces ioniques avec l'iode présent dans le milieu, tels que les composés organo-azotés ou organo-phosphorés. Les composés du type des iodures alcalins sont en fait les éléments préférés puisqu'ils sont presque systématiquement employés dans les procédés de carbonylation. En effet, l'addition de ces composés a permis de baisser la teneur en eau dans le réacteur et donc de rendre plus compétitifs de tels procédés par rapport aux précédents décrits.

Un autre type de solution a été proposé, consistant à introduire du monoxyde de carbone dans la zone de vaporisation partielle du mélange réactionnel, de telle sorte que la pression partielle en ce gaz dans ladite zone soit d'au plus 2 bar. Le monoxyde de carbone est introduit sous la forme d'un gaz pur ou dilué que l'on fait barboter dans la fraction liquide se trouvant dans le pied de l'appareil où est effectuée la séparation par vaporisation partielle du mélange réactionnel. Un tel procédé est décrit dans EP-A-441 260. Ce procédé présente plusieurs inconvénients. Tout d'abord, il se produit une perte inévitable de monoxyde de carbone. En effet, pour maintenir une pression partielle en monoxyde de carbone de cet ordre dans le flash, où la pression totale est par ailleurs relativement faible, il est nécessaire de mettre en oeuvre des débits de monoxyde de carbone très importants. De plus la solubilisation du monoxyde de carbone ne peut être obtenue de manière efficace qu'en présence d'une agitation énergique. Or un flash ne comprend pas de tels moyens. Ceci a pour conséquence que la majeure partie du flux de monoxyde de carbone va s'échapper avec la fraction vaporisée du mélange réactionnel. A ce stade, il existe deux alternatives. Soit le gaz est détruit, ce qui n'est pas admissible car la quantité perdue est très élevée et ce gaz est l'un des réactifs de la réaction de carbonylation. Soit le gaz est recyclé, mais ceci occasionne un surcoût non négligeable car il est nécessaire de séparer ce gaz des sous-produits gazeux de la réaction, qui sont notamment l'hydrogène, le méthane et le dioxyde de carbone. En outre, le fait de mettre en oeuvre des débits aussi importants peut avoir des conséquences au niveau de l'installation en elle-même, notamment en ce qui concerne la taille des appareillages.

La présente invention a donc pour objet un procédé de préparation d'acides carboxyliques par carbonylation en présence d'un catalyseur à base de rhodium, dans lequel la stabilisation du catalyseur est réalisée de façon simple et efficace.

Le procédé selon l'invention peut en outre permettre de diminuer la teneur en agents stabilisants le catalyseur ainsi que la teneur en eau.

De plus le procédé selon l'invention évite toute perte inutile en monoxyde de carbone.

Ainsi, le procédé de préparation d'acides carboxyliques selon l'invention consiste à effectuer, dans une première zone, la réaction en phase liquide, en présence d'un catalyseur à base de rhodium, puis dans une seconde zone, à vaporiser partiellement le mélange réactionnel obtenu ; la fraction vaporisée, comprenant l'acide carboxylique produit, est purifiée ultérieurement et la fraction liquide non vaporisée, comprenant le catalyseur, est recyclée à la première zone. La caractéristique du procédé est que l'on met en contact la fraction liquide non vaporisée issue de la seconde zone avec du monoxyde de carbone de telle sorte que ce composé ne retoume pas dans la seconde zone.

Mais d'autres avantages et caractéristiques apparaîtront plus clairement à la lecture de la description et des figures qui vont suivre, pour lesquelles :
- la figure 1 représente un premier mode de réalisation dans lequel on met en contact la fraction non vaporisée avec du monoxyde de carbone sous la forme d'un gaz.
- la figure 2 représente un second mode de réalisation dans lequel on dissout du monoxyde de carbone dans une partie de la fraction liquide non vaporisée issue du flash.
- la figure 3 représente une variante du mode précédent dans lequel on dissout du monoxyde de carbone dans une partie de la fraction liquide non vaporisée issue du flash, ayant été au préalable traitée sur une résine échangeuse d'ions.

Le procédé selon l'invention peut donc être mis en oeuvre pour la préparation des acides carboxyliques par carbonylation.

Dans ce qui va suivre, les teneurs sont exprimées en pourcentage en poids sauf indicaiton contraire.

Le réactif employé dans ce type réaction est en général choisi parmi les alcools. Parmi les alcools convenables à la mise en oeuvre de la réaction, on peut citer les alcools, saturés, mono ou dihydroxylés, présentant un à dix atomes de carbone. A titre d'exemples de tels composés, on peut citer notamment le méthanol, l'éthanol, le propanol, le butanol, le 1,4-butanediol. Les alcools monohydroxylés sont préférés.

Il est important de noter que l'alcool peut être présent dans le milieu réactionnel en tant que tel ou sous forme masquée. En effet, il peut se trouver indifféremment sous la forme d'un dérivé halogéné et/ou d'un éther et/ou d'un ester obtenu par réaction entre ledit alcool et l'acide carboxylique présent.

La réaction est réalisée en présence d'un système catalytique comprenant d'une part une espèce soluble de rhodium et d'autre part, un promoteur halogéné.

Généralement, les composés à base de rhodium utilisés sont choisis parmi les complexes de coordination de ce métal, solubles dans le milieu, dans les conditions de réaction. Plus particulièrement on utilise des complexes de coordination dont les ligands sont d'une part le monoxyde de carbone et d'autre part un halogène qui est plus particulièrement l'iode. Bien entendu, il est possible de mettre en oeuvre des complexes solubles à base de ligands organiques. A titre de composés à base de rhodium, on pourra notamment se référer au brevet US 3 769 329 qui en donne une liste indicative.

En ce qui concerne le promoteur, celui-ci correspond plus particulièrement à la forme halogénée de l'alcool précité. De préférence, l'halogène est l'iode.

La teneur en promoteur halogéné est en général maintenue entre 5 et 20 %.

Par ailleurs, la réaction de carbonylation est effectuée en présence de l'ester correspondant à l'alcool et à l'acide fabriqué.

Plus particulièrement, la teneur en ester est comprise entre 0,5 et 5 %.

La réaction de carbonylation est en outre mise en oeuvre en présence d'eau. La teneur en eau peut varier dans de larges limites et être notamment comprise entre 0 exclu et 20 %. Cependant, de préférence elle est inférieure à 10%.

Le mélange réactionnel comprend en outre un agent stabilisant le catalyseur. Cet agent est en général choisi parmi les iodures de métaux alcalins, tels que l'iodure de potassium, de lithium, bien que des iodures de composés organiques soient envisageables.

Généralement la teneur en cet agent est comprise entre 2 et 20%. En mettant en oeuvre le procédé selon l'invention, il est possible de baisser la quantité d'iodure nécessaire. Ainsi, il est envisageable d'effectuer la réaction en présence d'une quantité d'iodure aussi faible que de l'ordre de 1%.

Enfin, le solvant de la réaction est d'une façon avantageuse l'acide carboxylique que l'on souhaite fabriquer.

La réaction de carbonylation est mise en oeuvre à une température comprise entre 150 et 250 °C.

La pression totale dans le réacteur est habituellement comprise entre 1 et 100 bar absolus.

La pression partielle en monoxyde de carbone dans le réacteur varie plus particulièrement entre 1 et 50 bar absolus.

Le monoxyde de carbone peut être introduit dans le réacteur sous une forme pure ou diluée dans un gaz, tel que l'hydrogène, le méthane ou encore l'azote.

Le mélange réactionnel est ensuite traité, de façon continue, dans la zone de séparation de l'acide formé, dans laquelle une fraction dudit mélange réactionnel est vaporisée. Dans cette partie, la pression totale est plus réduite que celle du réacteur. Elle est en général comprise entre 1 et 20 bar absolus.

L'opération peut avoir lieu sans apport de chaleur au flash (conditions adiabatiques) ou bien avec apport de chaleur. Selon une variante préférée, la vaporisation partielle est effectuée dans un flash adiabatique.

Ainsi que cela a été mentionné auparavant, la fraction vaporisée comprend la production en acide carboxylique, mais aussi les réactifs et sous-produits de la réaction. Cette fraction vaporisée est envoyée dans la zone de purification de l'acide, qui comprend de façon classique plusieurs colonnes de distillation dans lesquelles on purifie l'acide produit.

La fraction liquide non vaporisée, comprenant notamment le catalyseur est récupérée en pied du flash pour être recyclée dans le réacteur.

Le procédé de carbonylation selon l'invention consiste donc à mettre en contact ladite fraction non vaporisée avec du monoxyde de carbone de telle sorte que ce composé ne retourne pas dans la seconde zone, celle de vaporisation partielle du mélange réactionnel. Lorsqu'il est dit que l'introduction du monoxyde de carbone est telle que ce composé ne retourne pas dans la seconde zone, on entend mettre en oeuvre des moyens évitant le dégazage de ce composé directement vers le flash.

Sans vouloir être limité par une quelconque théorie, il a été trouvé que le catalyseur avait tendance à former des espèces inactives, selon un processus relativement lent et réversible. Ces espèces entraînent la formation de précipités après plusieurs passages dans la boucle de recyclage de la zone de séparation à celle de réaction. Or l'introduction de monoxyde de carbone dans cette partie du procédé, sans avoir de retour dudit composé dans la zone de flash, permet de réduire la concentration de ces espèces inactives. Par conséquent la formation de précipité est, d'une manière inattendue, évitée et le catalyseur est réactivé.

Selon une première variante de l'invention, le monoxyde de carbone est introduit sous la forme d'un gaz.

Afin de ne pas avoir de retour dudit composé vers le flash, l'introduction a lieu en aval de la pompe grâce à laquelle la fraction liquide non vaporisée est recyclée au réacteur.

Le monoxyde de carbone peut être employé sous une forme pure ou bien comprendre d'autres gaz comme de l'hydrogène, du méthane, de l'azote. De préférence, le monoxyde de carbone mis en oeuvre présente une pureté suffisante pour éviter l'accumulation de trop de gaz qui ne sont pas actifs pour la réaction de carbonylation.

La pression partielle en monoxyde de carbone employée varie dans de larges limites.

La fraction non vaporisée peut par ailleurs subir tout type de traitement, dans le but notamment de purifier celle-ci, tel que par exemple un traitement pour éliminer les métaux de corrosion, qui sera détaillé plus loin.

Selon une seconde variante du présent procédé, la fraction non vaporisée du mélange réactionnel, issue du flash, est mise en contact avec un flux liquide comprenant du monoxyde de carbone à l'état dissous, avant d'être retournée au réacteur.

Cette variante est avantageuse en ce sens qu'introduire un liquide dans lequel le monoxyde de carbone est dissous permet de rendre instantanément actif ce composé.

Le monoxyde de carbone peut être là encore employé sous une forme pure ou bien comprendre d'autres gaz comme de l'hydrogène, du méthane, de l'azote. Ce qui a été indiqué à ce sujet lors de la première variante reste valable dans ce cas.

La quantité de monoxyde de carbone introduite est au plus égale à la limite de la solubilité de ce gaz dans le liquide employé, selon les conditions de température et de pression. Plus particulièrement la quantité de monoxyde de carbone dissous est comprise entre 0 exclu et 10 %, cette teneur étant liée à la température et la pression du liquide.

Le flux liquide dans lequel le monoxyde de carbone est dissous, est choisi de manière à être compatible avec le mélange réactionnel de carbonylation.

Selon un premier mode de réalisation, le flux liquide dans lequel le monoxyde de carbone est dissous est constitué par l'acide carboxylique et/ou tout autre réactif employé durant la carbonylation. L'acide carboxylique, ou tout autre réactif, peut être mis en oeuvre sous une forme pure ou bien provenir, d'une façon avantageuse, de la zone de purification située en aval.

Selon un second mode de réalisation, le monoxyde de carbone est dissous dans une solution comprenant en outre le catalyseur. Selon ce mode de réalisation, au moins une partie de la fraction liquide non vaporisée provenant de la zone de séparation, est dérivée de la boucle de recyclage pour être mise en contact avec le monoxyde de carbone. Le flux liquide ainsi traité est ensuite rassemblé avec l'autre partie de la fraction liquide sortant du flash.

Ce mode de réalisation est particulièrement avantageux en ce sens qu'une partie du catalyseur est traitée avec le monoxyde de carbone, permettant ainsi sa réactivation.

Selon une mise en oeuvre particulière de ce second mode de réalisation, la partie de la fraction liquide dans laquelle est solubilisé le monoxyde de carbone est au préalable traitée sur une résine échangeuse d'ions pour en éliminer les métaux de corrosion. Ceci est réalisé par tout moyen connu de l'homme du métier.

Ainsi, on peut utiliser plus particulièrement des résines du type acide fort, sous une forme hydrogène. Ces résines peuvent être sous la forme de gel ou encore macroréticulées.

La mise en contact peut être classiquement effectuée en lit fixe ou en lit fluidisé.

La température à laquelle on réalise le traitement doit être bien sur adaptée à la résistance de la résine. En général, elle est comprise entre la température ambiante et 100°C.

Quelle que soit la solution dans laquelle est dissous le monoxyde de carbone, l'opération de solubilisation du gaz a lieu dans un appareil sous une forte agitation. N'importe quel moyen peut être mis en oeuvre pour obtenir un tel résultat, qu'il soit de nature mécanique ou non.

La température de mise en contact est comprise entre 25 et 200°C.

La pression est au moins égale à la pression régnant dans le réacteur. Plus particulièrement, la pression est comprise entre 1 et 100 bar absolus.

La solution comprenant le monoxyde de carbone dissous est introduite dans une partie de la boucle de recyclage de manière à éviter le dégazage du monoxyde de carbone et son entraînement vers le flash, de même qu'éviter la cavitation de la pompe grâce à laquelle la fraction liquide provenant du flash est retournée au réacteur. L'homme du métier est à même, avec ses propres connaissances de génie chimique de déterminer l'endroit approprié pour l'introduction de la solution comprenant le monoxyde de carbone dissous.

Il est à noter que le dégazage d'une partie du monoxyde de carbone vers le réacteur ne pose pas de problème particulier, car ce composé est consommé dans la réaction de carbonylation et n'est donc pas perdu avec les sous-produits gazeux.

Il est à noter que l'on ne sortirait pas du cadre de la présente invention en mettant en oeuvre une combinaison des deux variantes décrites auparavant, c'est-à-dire la mise en contact de la fraction non vaporisée avec du monoxyde de carbone dissous ou non dans un flux liquide.

Les figures vont maintenant être décrites.

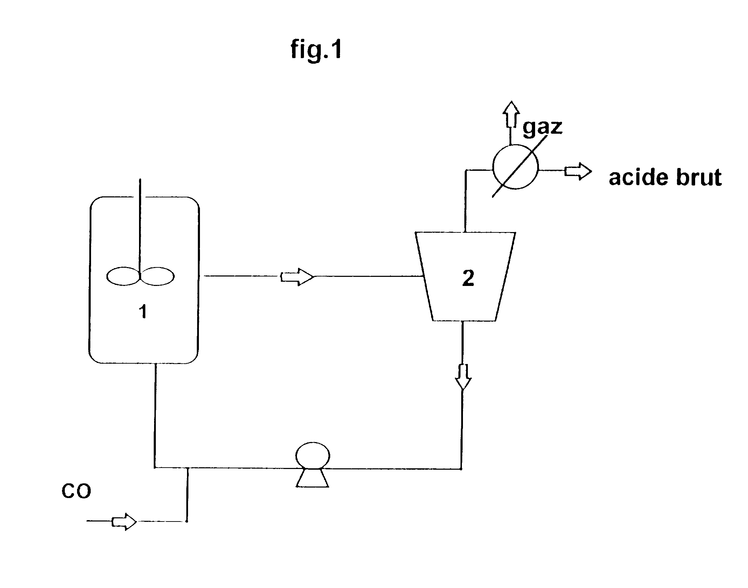

La figure 1 décrit un mode de réalisation de la première variante, dans lequel la fraction non vaporisée est mise en contact avec du monoxyde de carbone sous la forme de gaz. Ainsi, le monoxyde de carbone et l'alcool sont introduits dans le réacteur (1) comprenant le système catalytique, l'acide carboxylique, l'ester, l'eau et l'agent stabilisant. En sortie du réacteur, le mélange réactionnel est détendu au moyen d'une vanne qui ne figure pas sur le schéma, ce qui entraîne une vaporisation partielle du mélange et introduit dans le flash (2). La fraction vaporisée est condensée de façon à séparer l'acide carboxylique à purifier des produits incondensables que sont le monoxyde de carbone, et les sous-produits gazeux de la réaction. La fraction restant liquide est, elle, engagée dans la boucle de recyclage vers le réacteur. Le monoxyde de carbone est introduit en aval de la pompe employée pour recycler la fraction liquide.

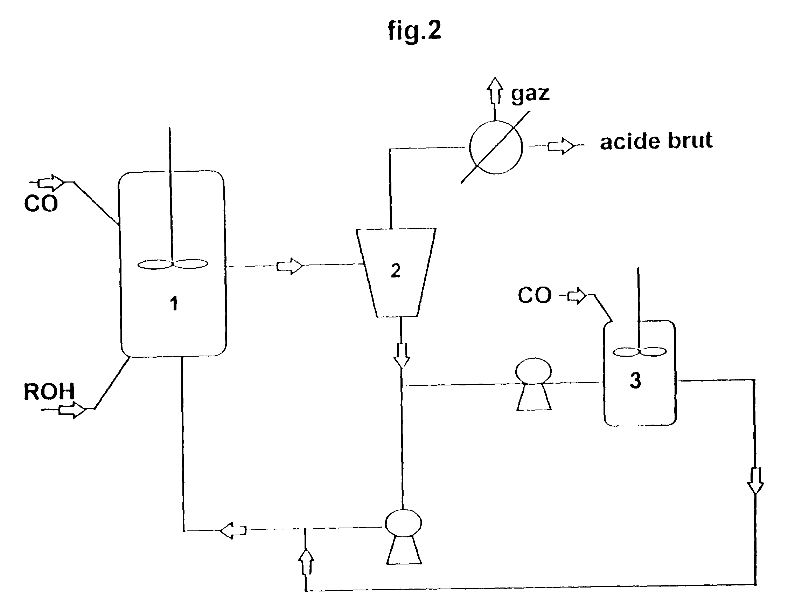

La figure 2 décrit un mode de réalisation de la seconde variante, dans lequel une partie de la fraction non vaporisée est mise en contact avec du monoxyde de carbone dissous dans un flux liquide. Ainsi, la réaction de carbonylation a lieu comme représenté dans la figure précédente. En sortie du réacteur, le mélange réactionnel est introduit dans le flash (2). La fraction vaporisée est condensée pour séparer l'acide carboxylique à purifier des produits incondensables. La fraction restant liquide est engagée dans la boucle de recyclage vers le réacteur. Un partie de ce flux est alors dérivée de la boucle de recyclage pour être introduite dans un réacteur (3) muni de moyens d'agitation et d'introduction de monoxyde de carbone. Une fois la solubilisation de ce gaz effectuée, le liquide enrichi en monoxyde de carbone est rassemblé au flux qui n'a pas été traité, après avoir été au préalable et si nécessaire, détendu de façon à retrouver une pression voisine de celle du réacteur, au moyen d'une vanne qui n'est pas représentée.

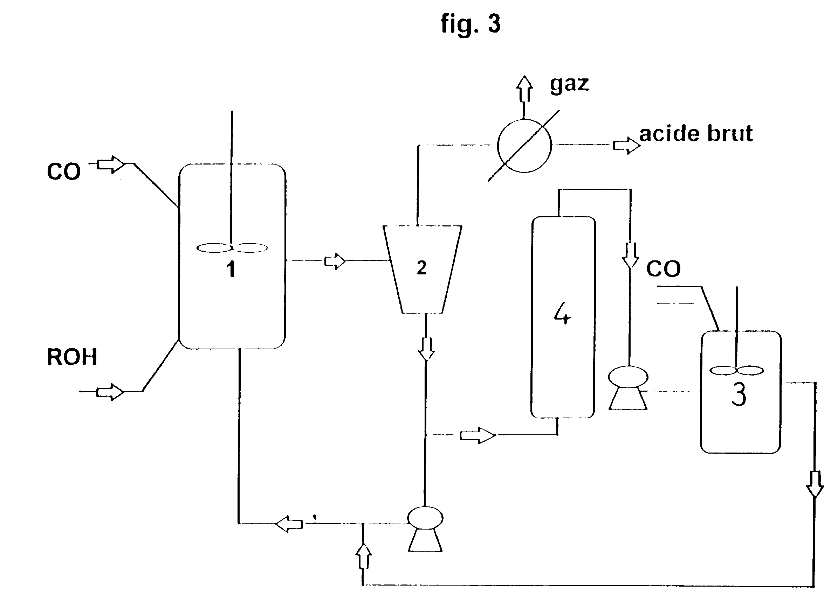

La figure 3 décrit une variante du mode décrit dans la figure précédente. La différence entre ces deux modes réside dans le fait que la fraction dérivée de la boucle de recyclage est traitée par passage dans une colonne (4) sur une résine échangeuse d'ions avant la mise en contact avec le monoxyde de carbone.

Sur chacune des deux dernières figures, la dérivation du flux à mettre en contact avec le monoxyde de carbone est effectuée en amont de la pompe permettant le recyclage du liquide vers le réacteur. On ne sortirait pas du cadre de la présente invention en effectuant cette dérivation an aval de cette même pompe.

D'une manière similaire, pour ces deux mêmes figures, le point de contact entre le liquide comprenant le monoxyde de carbone dissous et celui qui n'a pas été traité avec ce gaz, se situe en aval de la pompe précitée. Là encore, on ne sortirait pas du cadre de la présente invention en effectuant ceci en amont de la pompe.

Bien entendu, ces trois figures ne sont que des exemples de mise en oeuvre de l'invention et ne doivent pas être considérées comme des limitations de celle-ci.

## Revendications

1. Procédé de préparation d'acides carboxyliques dans lequel on effectue, dans une première zone la réaction en phase liquide entre monoxyde du carbone et un alcool, en présence d'un catalyseur à base de rhodium et un promoteur halogéné, puis dans lequel, dans une seconde zone, on vaporise partiellement le mélange réactionnel obtenu ; la fraction vaporisée, comprenant l'acide carboxylique produit est purifiée ultérieurement et la fraction liquide non vaporisée, comprenant le catalyseur est recyclée à la première zone, caractérisé en ce que l'on met en contact ladite fraction non vaporisée avec du monoxyde de carbone de telle sorte que ce composé ne retourne pas dans la seconde zone.

2. Procédé selon la revendication précédente, caractérisé en ce que l'on recycle à la première zone la fraction non vaporisée au moyen d'une pompe et que l'introduction du monoxyde de carbone a lieu en aval de ladite pompe.

3. Procédé selon la revendication 1, caractérisé en ce que l'on met en contact la fraction non vaporisée avec du monoxyde de carbone dissous dans un flux liquide.

4. Procédé selon la revendication précédente, caractérisé en ce que le flux liquide dans lequel le monoxyde de carbone est dissous est une solution compatible avec le mélange réactionnel.

5. Procédé selon l'une quelconque des revendications 3 à 4, caractérisé en ce que le flux liquide dans lequel le monoxyde de carbone est dissous comprend le catalyseur.

6. Procédé selon la revendication précédente, caractérisé en ce que le flux liquide est constitué par une partie de la fraction non vaporisée.

7. Procédé selon la revendication précédente, caractérisé en ce que ladite fraction non vaporisée est au préalable traitée sur une résine échangeuse d'ions.

8. Procédé selon l'une quelconque des revendications 3 à 7, caractérisé en ce que la quantité de monoxyde de carbone dissous varie de 0 exclu à 10 %, selon les conditions de température et de pression du liquide.

## Claims

1. Process for preparing carboxylic acids in which there is carried out, in a first zone, the reaction in liquid phase between carbon monoxide and an alcohol, in the presence of a catalyst based on rhodium and a halogenated promoter, then in which, in a second zone, the reaction mixture obtained is partially vaporized; the vaporized fraction containing the carboxylic acid produced is subsequently purified and the non-vaporized liquid fraction containing the catalyst is recycled to the first zone, characterised in that said non-vaporized fraction is placed in contact with carbon monoxide in such a way that this compound does not return into the second zone.

2. Process according to the previous claim, characterised in that the non-vaporized fraction is recycled to the first zone by means of a pump and that the introduction of the carbon monoxide takes place downstream of said pump.

3. Process according to claim 1, characterised in that the non-vaporized fraction is placed in contact with carbon monoxide dissolved in a liquid flux.

4. Process according to the previous claim, characterised in that the liquid flux in which the carbon monoxide is dissolved is a solution compatible with the reaction mixture.

5. Process according to any one of claims 3 to 4, characterised in that the liquid flux in which the carbon monoxide is dissolved contains the catalyst.

6. Process according to the previous claim, characterised in that the liquid flux is composed of a part of the non-vaporized fraction.

7. Process according to the previous claim, characterised in that said non-vaporized fraction is treated beforehand on an ion exchange resin.

8. Process according to any one of claims 3 to 7, characterised in that the quantity of dissolved carbon monoxide varies from 0 exclusive to 10%, according to the temperature and pressure conditions of the liquid.

## Patentansprüche

1. Verfahren zur Herstellung von Carbonsäuren, bei dem man in einer ersten Zone die Reaktion zwischen Kohlenmonoxid und einem Alkohol in flüssiger Phase in Gegenwart eines Katalysators auf der Basis von Rhodium und eines halogenhaltigen Promotors ausführt, bei dem man dann in einer zweiten Zone das erhaltene Reaktionsgemisch teilweise verdampft; die verdampfte Fraktion, die die erzeugte Carbonsäure umfaßt, wird später gereinigt, und die nicht verdampfte flüssige Fraktion, die den Katalysator umfaßt, wird in die erste Zone zurückgeführt, dadurch gekennzeichnet, daß man besagte nicht verdampfte Fraktion derart mit Kohlenmonoxid in Kontakt bringt, daß diese Verbindung nicht in die zweite Zone zurückkehrt.

2. Verfahren gemäß dem vorhergehenden Anspruch, dadurch gekennzeichnet, daß man die nicht verdampfte Fraktion mittels einer Pumpe in die erste Zone zurückführt und daß die Einleitung des Kohlenmonoxids nach besagter Pumpe stattfindet.

3. Verfahren gemäß Anspruch 1, dadurch gekennzeichnet, daß man die nicht verdampfte Fraktion mit Kohlenmonoxid in Kontakt bringt, das in einem Flüssigkeitsstrom gelöst ist.

4. Verfahren gemäß dem vorhergehenden Anspruch, dadurch gekennzeichnet, daß der Flüssigkeitsstrom, in dem das Kohlenmonoxid gelöst ist, eine mit dem Reaktionsgemisch verträgliche Lösung ist.

5. Verfahren gemäß einem der Ansprüche 3 bis 4, dadurch gekennzeichnet, daß der Flüssigkeitsstrom, in dem das Kohlenmonoxid gelöst ist, den Katalysator umfaßt.

6. Verfahren gemäß dem vorhergehenden Anspruch, dadurch gekennzeichnet, daß der Flüssigkeitsstrom aus einem Teil der nicht verdampften Fraktion besteht.

7. Verfahren gemäß dem vorhergehenden Anspruch, dadurch gekennzeichnet, daß besagte nicht verdampfte Fraktion vorher über einem lonenaustauscherharz behandelt wird.

8. Verfahren gemäß einem der Ansprüche 3 bis 7, dadurch gekennzeichnet, daß die Menge an gelöstem Kohlenmonoxid zwischen 0, ausgeschlossen, und 10% je nach den Temperatur- und Druckbedingungen der Flüssigkeit variiert.
